# EUROPÄISCHE PATENTSCHRIFT

(11) **EP 1 479 388 B1**
(45) Veröffentlichungstag und Bekanntmachung des Hinweises auf die Patenterteilung: **07.12.2005**
(21) Anmeldenummer: 04011905.9
(22) Anmeldetag: 19.05.2004
(51) Int. Cl.: A61K 31/70, A61K 47/10, A61K 47/18, A61P 35/00

(54) **Gebrauchsfertige Gemcitabin-Lösungskonzentrate**
Ready to use gemcitabin solution concentrates
Concentrés de solution de gemcitabine prêtes à l'emploi

(30) Priorität: 21.05.2003 DE 10323278
(43) Veröffentlichungstag der Anmeldung: 24.11.2004
(73) Patentinhaber: STADA ARZNEIMITTEL AG, 61118 Bad Vilbel (DE)
(72) Erfinder: Schridde, Edgar, 30451 Hannover (DE); Merbach, Bernd, Dr., 30938 Burgwedel (DE); Gimmel, Stefan-Peter, Dr., 30655 Hannover (DE)
(74) Vertreter: Hamm, Volker

(56) Entgegenhaltungen:
- WO-A-99/52514
- US-A1- 2002 169 154

## Beschreibung

Die vorliegende Erfindung betrifft Gemcitabin umfassende pharmazeutische Zusammensetzungen in der Form von gebrauchsfertigen Lösungskonzentraten.

Gemcitabin (2'-Deoxy-2',2'-difluorcytidin; 1-(4-Amino-2-oxo-1H-pyrimidin-1-yl)-2-deoxy-2,2-difluorribose; dFdC; CAS Nr. 95058-81-4; C₉H₁₁F₂N₃O₄, Mᵣ 263,2) ist eine im Arzneibuch offiziell monographierte Substanz (Official Monographs, USP 27, 1^{st} Supplement USP-NF, page 3060-61 bzgl. "Gemcitabine Hydrochloride" and "Gemcitabine for Injection") und hat die chemische Struktur 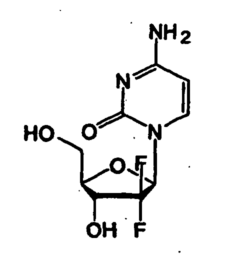 Gemcitabin wurde erstmals in US 4,526,988 beschrieben und kann zur Behandlung von viralen Infektionen oder in der immunosuppressiven Therapie von Autoimmunerkrankungen eingesetzt werden. Die anti-neoplastische Wirksamkeit des Gemcitabins ist in US 5,464,826 offenbart. Es wird beispielsweise allein, aber auch in Kombination mit anderen Zytostatika wie etwa Cisplatin in der Behandlung des lokal fortgeschrittenen oder metastasierten, nichtkleinzelligen Bronchialkarzinoms sowie des fortgeschrittenen Adeno- oder Cysadenokarzinoms des exokrinen Pankreas therapeutisch eingesetzt. Die empfohlene Dosis in der Gemcitabin-Therapie beträgt 1 g / qm Körperoberfläche. Wie andere Nukleosidanaloga kann auch Gemcitabin zytostatisch bei der therapeutischen Behandlung verschiedenster Krebsarten wie z.B. der lymphatischen oder myeloischen Leukämie eingesetzt werden. Dabei erfolgt die Verabreichung von Gemcitabin in der Therapie der verschiedensten Krebserkrankungen intravenös, wodurch es erforderlich wird, den Wirkstoff in Form einer Lösung zur Verfügung zu stellen.

Die zur parenteralen Verabreichung benötigten Gemcitabin-Zubereitungen sind zur Zeit nur in Form von Lyophilisaten verfügbar, die vor der Verabreichung an den Patienten rekonstituiert werden müssen. Die Verwendung von solchen gefriergetrockneten Zubereitungen birgt aber wesentliche Nachteile. Zum einen ist das Herstellungsverfahren der Lyophilisate kompliziert und teuer, zum anderen stellt die Rekonstitution zusätzliche Arbeitsschritte dar und bedeutet für das damit befasste Personal unerwünschte Risiken. Insbesondere kann es bei der Rekonstituierung der Arzneimittellösungen aus einer Trockensubstanz zum sogenannten "Spray-Back-Effect" kommen, durch den es zu einer weiteren Kontamination und Gefährdung des Personals kommen kann. Demgemäss muss sowohl bei der Herstellung des Lyophilisates, als auch bei seiner Rekonstitution jede Kontamination von Personal oder Inventar mit dem hochwirksamen Zytostatikum vermieden werden. Zudem kann es auch durch andere Fehler in der Handhabung dieser Lyophilisate zu schwerwiegenden Problemen bei der Behandlung mit Gemcitabin kommen, wie z.B. zu einer Abweichung der Wirkstoffkonzentration oder einer mikrobiellen Kontamination der aus dem Lyophilisat hergestellten Lösung.

Zudem weisen die bekannten, aus Lyophilisaten rekonstituierten Gemcitabin-Zubereitungen den Nachteil auf, dass deren Gemcitabin-Konzentration auf 38 mg/ml begrenzt ist und diese auch nur bei einem pH-Wert von 2,7 bis 3,3 realisiert werden kann. Dies hängt bekanntlich damit zusammen, dass die Löslichkeit des Gemcitabins in Wasser mit zunehmendem pH-Wert abnimmt, so dass dessen Löslichkeit in einem pH-Bereich von 2,7 bis 3,3 ca. 38 mg/ml, bei einem pH-Wert von 5 16,0 mg/ml, bei einem pH-Wert von 7 15,3 mg/ml und bei einem pH-Wert von 9 15,8 mg/ml beträgt. Um das zum Lyophilisat gefrierzutrocknende Lösungsvolumen zu verringern, wird deshalb der Wirkstoff in Wasser mit einem pH-Bereich von 2,7 bis 3,3 gelöst. Laut Gebrauchs- bzw. Fachinformation des GEMZAR®-Lyophilisats wird bei den aus dem GEMZAR®-Lyophilisat mit Hilfe von NaCl-Lösung rekonstituierten Lösungen ein pH-Wert in genau diesem Bereich maximaler Löslichkeit (pH 2,7 bis 3,3) erhalten.

Ferner ist von Nachteil, dass zur Einhaltung der empfohlenen Dosis von 1 g / qm Körperoberfläche in der Gemcitabin-Therapie große Lösungsvolumina verwendet werden müssen, um aus den Lyophilisaten Infusionslösungen zu rekonstituieren. Beispielsweise wird bei einer durchschnittlichen Dosis von 1,8 g / 1,8 qm Körperoberfläche ein rekonstituiertes Lösungsvolumen von 47 ml mit 250 bis 500 ml einer Trägerlösung verdünnt. Aus Gründen einer sicheren Handhabung wäre es von Vorteil, mit möglichst kleinen Lösungsvolumina zu hantieren. Ebenfalls wäre es aus Betriebskostengründen von Vorteil, für die Lagerung von Arzneimitteln möglichst kleine Packungsgrößen (z. B. Durchstechflaschen) zu verwenden. Diese zusätzlichen Vorteile bietet ein gebrauchsfertiges Lösungskonzentrat.

Aufgabe der vorliegenden Erfindung ist es damit, gebrauchsfertige Gemcitabin-Lösungskonzentrate zur Verfügung zu stellen, die nicht die zuvor diskutierten Risiken und Nachteile der bekannten Darreichungsformen haben.

In den der Erfindung zugrundeliegenden Experimenten wurde überraschend gefunden, dass die Löslichkeit des Gemcitabins in wässrigen Lösungskonzentraten selbst bei hohen pH-Werten signifikant erhöht werden konnte, indem das Gemcitabin in einer Mischung aus Wasser und mindestens einem weiteren physiologisch annehmbaren Lösungsmittel bzw. Solubilisierungsmittel gelöst wurde.

Ferner wurde in den der Erfindung zugrundeliegenden Experimenten überraschend gefunden, dass erfindungsgemäße Gemcitabin-Lösungskonzentraite mit einem pH-Wert von ca. 3,5 bis ca. 10,0 unerwartet hohe Lagerstabilitäten bei verschiedenen Lagertemperaturen zeigten.

Gemäß einer besonders bevorzugten Ausführungsform umfasst das erfindungsgemäße Lösungskonzentrat eine Gemcitabin-Konzentration von 50 mg/ml oder 80 mg/ml sowie einen Ethanolanteil von 50 Vol.-% bzw. 60 Vol.-% und hat einen pH-Wert von ca. 8,0.

Die erfindungsgemäßen Lösungskonzentrate umfassen zwischen 16,0 mg und 110,0 mg Gemcitabin pro ml Lösungsmittel. Vorzugsweise beträgt die Konzentration des Gemcitabins 50 mg/ml oder 80 mg/ml.

Die erfindungsgemäßen Lösungskonzentrate können die freie Gemcitabin-Base oder ein physiologisch annehmbares Säureadditionssalz davon umfassen. Bevorzugt wird die freie Gemcitabin-Base, besonders bevorzugt das Säureadditionssalz mit einer anorganischen Säure, insbesondere bevorzugt Gemcitabinhydrochlorid zur Herstellung der erfindungsgemäßen Lösungskonzentrate verwendet.

Als geeignete Lösungsmittelgemische für erfindungsgemäße Lösungskonzentrate sind beispielsweise Mischungen von Wasser mit Ethanol, Glycerin, 1,2-Propandiol (Propylenglykol), Polyethylenglykol 200-600, Benzylalkohol, Trimethylenglykol, 1,3-Butylenglykol, 2,3-Butylenglykol, Ethylacetat, Ethyllactat, Glykofurol (Tetraglykol), Solketal, physiologisch annehmbaren Cyclodextrinen (α-, β-, γ-Cyclodextrinen) sowie deren Alkyl- und/oder Aryl-substituierten Derivaten und/oder Harnstoff als lösungsvermittelnde und tonisierende Hilfsstoffe zu nennen. Vorzugsweise wird eine Mischung von Wasser mit Ethanol, Polyethylenglykol 200-600 und/oder 1,2-Propandiol (Propylenglykol) verwendet. Besonders bevorzugt ist ein Gemisch aus Wasser, Ethanol und/oder Polyethylenglykol 300-400. Insbesondere bevorzugt ist eine Mischung aus Wasser und Ethanol. Die Mischungen können dabei in verschiedenen Verhältnissen verwendet werden.

Gemäß einer bevorzugten Ausführungsform liegt der pH-Wert der erfindungsgemäßen Lösungskonzentrate in einem Bereich von ca. 3,5 bis ca. 10,0. Gemäß einer Ausführungsform zeigen die erfindungsgemäßen Gemcitabin-Lösungskonzentrate einen pH-Wert von 4,0 bis 5,0; gemäß einer weiteren erfindungsgemäßen Ausführungsform zwischen 7,0 und 10,0; besonders bevorzugt 7,0 bis 9,0. Gemäß einer weiteren, bevorzugten erfindungsgemäßen Ausführungsform stimmt der pH-Wert der Lösungskonzentrate mit dem physiologischen Gewebe- und Blut-pH-Wert von 7,35 bis 7,55 überein. Bei einer besonders bevorzugten erfindungsgemäßen Ausführungsform liegt der pH-Wert des Konzentrats in einem Bereich von 7,8 bis 8,2.

Die Einstellung des pH-Wertes eines erfindungsgemäßen Lösungskonzentrates kann durch Mischen eines geeigneten Verhältnisses der Gemcitabin-Base mit einem physiologisch annehmbaren Säureadditionssalz davon, vorzugsweise mit Gemcitabinhydrochlorid, erfolgen.

Erfindungsgemäß ist es aber auch möglich, den pH-Wert mit mindestens einem physiologisch annehmbaren Ansäuerungs- und/oder Alkalisierungsmittel einzustellen. Beispielsweise sind hierfür geeignet anorganische Säuren und Basen, wie z. B. Salzsäure, Schwefelsäure, schweflige Säure, Salpetersäure, salpetrige Säure, Phosphorsäure, phosphorige Säure, Kohlensäure, Natriumhydroxid, Kaliumhydroxid, Calciumhydroxid und Magnesiumhydroxid; Alkali- und Erdalkalisalze sowie Alkalihydrogen- und Erdalkalihydrogensalze der anorganischen Oxosäuren des Phosphors, Schwefels, Kohlenstoffs und Stickstoffs wie z. B. Natriumphosphat und dessen Hydrate, Natriumhydrogenphosphat und dessen Hydrate, Dinatriumhydrogenphosphat und dessen Hydrate, Dinatriumsulfat, Natriumhydrogensulfat, Natriumsulfit, Calciumsulfit, Magnesiumsulfit, Calciumhydrogencarbonat, Natriumcarbonat, Natriumhydrogencarbonat, Natriumnitrat, Natriumnitrit, Calciumnitrit, Magnesiumnitrat und Magnesiumnitrit; Salze des Chlors wie z. B. Natriumchlorid, Calciumchlorid und Magnesiumchlorid; organische Basen und Säuren sowie Alkali- und Erdalkalisalze davon wie z.B Ameisensäure, Essigsäure, Propionsäure, Milchsäure, Oxalsäure, Malonsäure, Maleinsäure, Weinsäure, Zitronensäure, Brenztraubensäure, Benzoesäure, Methansulfonsäure, Ethansulfonsäure, *para-*Toluol-Sulfonsäure, Salicylsäure, Ascorbinsäure, Tris(hydroxymethyl-)aminomethan (2-Amino-2-(hydroxymethyl)-1,3-propandiol; Trometamol; TRIS), 1-Desoxy-(methylamino)-D-glucitol (N-Methylglucamin; Meglumin); Alkali- und Erdalkalisalze von organischen Basen und Säuren wie etwa Natriumacetat; sowie Mischungen davon.

Vorzugsweise wird der pH-Wert der erfindungsgemäßen Lösungskonzentrate eingestellt mit Salzsäure, Phosphorsäure, Schwefelsäure, Natriumhydroxid, Natriumphosphat und dessen Hydraten, Natriumhydrogenphosphat und dessen Hydraten, Dinatriumhydrogenphosphat und dessen Hydraten, Essigsäure, Milchsäure, Zitronensäure, Methansulfonsäure, Ethansulfonsäure, Tris(hydroxymethyl-)aminomethan (Trometamol; TRIS) oder 1-Desoxy-(methylamino)-D-glucitol (N-Methylglucamin; Meglumin), insbesondere bevorzugt mit Natriumhydroxid, Salzsäure, Tris(hydroxymethyl-)aminomethan (Trometamol; TRIS) oder 1-Desoxy-(methylamino)-D-glucitol (N-Methylglucamin; Meglumin) eingestellt.

Die Einstellung und/oder Stabilisierung des pH-Wertes kann auch durch einen aus einem aus physiologisch annehmbaren Ansäuerungs- und/oder Alkalisierungsmittel gebildeten Puffer erfolgen. Besonders bevorzugt sind Puffersysteme, wobei der pK-Wert von mindestens einer funktionellen Gruppe der den Puffer bildenden Pufferbase oder Puffersäure innerhalb des Bereiches von pK 2,49 bis 11,01 liegt. Besonders bevorzugte Puffer zur Einstellung des pH-Wertes der erfindungsgemäßen Lösungskonzentrate sind Natriumacetat, Tris(hydroxymethyl-)aminomethan (Trometamol; TRIS), 1-Desoxy(methylamino)-D-glucitol (N-Methylglucamin; Meglumin) und Dinatriumhydrogenphosphat oder Mischungen davon.

Die Pufferkonzentration in den erfindungsgemäßen Lösungskonzentraten liegt zwischen 0,001 g bis 100 g an Pufferkomponente, bevorzugt zwischen 0,05 g bis 20 g an Pufferkomponente und besonders bevorzugt zwischen 0,1 bis 10 g an Pufferkomponente jeweils bezogen auf 1 g Gemcitabin.

Die erfindungsgemäßen Lösungskonzentrate umfassen gegebenenfalls zusätzlich mindestens einen tonisierenden Hilfsstoff, mindestens einen konservierenden Hilfsstoff und/oder mindestens ein Antioxidationsmittel.

Als Tonizitätsmittel und lösungsvermittelnde Hilfsstoffe für erfindungsgemäße Lösungskonzentrate können beispielsweise verwendet werden physiologisch annehmbare anorganische Alkali- oder Erdalkalisalze wie z. B. Natriumchlorid, Calciumchlorid, Magnesiumchlorid, Natriumsulfat, Natriumcarbonat und Calciumhydrogencarbonat; physiologisch annehmbare organische Salze wie z. B. Natriumlactat; physiologisch annehmbare Kohlenhydrate wie z. B. physiologisch annehmbare Cyclodextrine (α-, β-, γ-Cyclodextrine) sowie deren Alkyl- und/oder Aryl-substituierte Derivate, Glucose, Fructose, Sorbitol, Mannitol, Galactose, Inositol, Maltitol, Lactose, Trehalose, Maltose, Saccharose, Dextran 1, Dextran 10, Dextran 40, Dextran 70, Stärke und Hydroxyethylstärke; physiologisch annehmbare Aminosäuren, Peptide oder Proteine wie etwa Glycin, Albumin und Gelatine; sowie Mischungen davon.

Bevorzugte Tonizitätsmittel sind Natriumchlorid, Calciumchlorid, Glucose, Mannitol und Lactose, insbesondere bevorzugt sind Natriumchlorid, Glucose und Mannitol.

Als Konservierungsmittel für erfindungsgemäße Lösungskonzentrate können beispielsweise verwendet werden Chlorcresol, Benzylalkohol, Ester der *p*-Hydroxybenzoesäure wie etwa Propylparaben, Ethylparaben und Methylparaben.

Bevorzugte Konservierungsmittel sind Benzylalkohol, Propylparaben und Methylparaben, insbesondere bevorzugt ist Benzylalkohol.

Als Antioxidationsmittel für die erfindungsgemäßen Lösungen können beispielsweise sauerstoffreduzierende, wie etwa Natriummetabisulfit und Natriumsulfit, oder Übergangsmetallionenbindende, komplex- (chelat-) bildende Antioxidantien, wie z.B. Natriumedetat, verwendet werden.

Zur Herstellung einer Infusionslösung wird das erfindungsgemäße Lösungskonzentrat mit der erforderlichen Menge einer Trägerlösung, wie z. B. 0,9%iger Natriumchloridlösung oder 5%iger Glucoselösung, verdünnt. Die nachfolgenden Beispiele verdeutlichen die Erfindung.

### Beispiele 1-2

Zur Bestimmung der Stabilität, der aus dem Stand Technik (*Journal of Pharmaceutical Sciences* **2000**, *89,* (7), *885-891*) bekannten, aus Trockensubstanz hergestellten Gemcitabin-Lösungen (Beispiel 1), wurden wässrige Lösungen (c = 40 mg/ml Gemcitabin, pH 3,2; 3,25 mg/ml Natriumacetat; Salzsäure, Natronlauge) hergestellt, und der Wirkstoffgehalt zeitaufgelöst mittels HPLC bestimmt. Eine solche Zusammensetzung ist annähernd vergleichbar mit einem herkömmlichen, aus GEMZAR®-Lyophilisat vorschriftsmäßig rekonstituierten Gemcitabinkonzentrat.

Laut Fachinformation zum Produkt GEMZAR®-Lyophilisat ergeben sich bei dessen Rekonstitution Lösungen mit pH-Werten im Bereich von 2,7 - 3,3. Aus dem Stand der Technik war bekannt, dass diese rekonstituierten Lösungen nicht lagerfähig waren, sondern unmittelbar an den Patienten verabreicht werden mussten. Mit den folgenden Beispielen konnte eindrucksvoll gezeigt werden, dass die erfindungsgemäßen Gemcitabin-Lösungskonzentrate eine erheblich verbesserte Lagerstabilität zeigen und somit eine Verwendung von Ready-to-Use-Lösungen in der therapeutischen Praxis erlauben.

Aus den mittels HPLC erhaltenen Stabilitätsdaten ließ sich für jede der angegebenen Lagertemperaturen die Zeitdauer t₉₅ regressiv interpolieren, innerhalb der ein pharmazeutisch geforderter minimaler Gehalt von 95% des initialen Wirkstoffgehaltes infolge Zersetzung erreicht wird.

**Tabelle 1:**

| Vergleich der Stabilität einer aus dem Stand der Technik (Bsp. 1) bekannten mit einer exemplarischen erfindungsgemäßen (Bsp. 2) Gemcitabin-Lösung. | | | |
|---|---|---|---|
| | Bsp.: | 1 | 2 |
| Gemcitabin (mg/ml) | | 40 | 40 |
| Natronlauge 1 N / Salzsäure 1 N | | q.s. | q.s. |
| Natriumazetat (mg/ml) | | 3,25 | - |
| Ethanol (in Vol%) | | - | 50 |
| Wasser für Injektionszwecke | | q.s. | q.s. |
| | pH-Wert | 3,2 | 8,2 |
| 25°C | Start | 100,0 | 100,0 |
| Gehalt (rel%) | 3 mon | 95,38 | 99,70 |
| | 6 mon | 83,70 | 99,61 |
| t₉₅ (Tage; 25°C) | | 68 | 2292 |
| 40°C | Start | 100,0 | 100,0 |
| Gehalt (rel%) | 1 mon | 89,41 | 99,77 |
| | 2 mon | 78,30 | 99,51 |
| | 3 mon | 62,05 | 99,28 |
| t₉₅ (Tage; 40°C) | | 15 | 620 |
| 60°C | Start | 100,0 | 100,0 |
| Gehalt (rel%) | 1 w | 79,21 | 99,72 |
| | 2 w | 56,06 | 99,51 |
| | 3 w | 20,05 | 99,30 |
| t₉₅ (Tage; 60°C) | | 2,2 | 151 |
| (mon = 30 Tage, w = 7 Tage) | | | |

Ein Vergleich der t₉₅-Werte in Tabelle 1 macht die Leistungsfähigkeit der vorliegenden Erfindung eindrucksvoll deutlich. So wird z.B. durch pH-Änderung von pH ca. 3 auf pH ca. 8 eine Steigerung der Stabilität um das 30 bis 70fache gegenüber dem vorbekannten Stand der Technik (o.g. Publikation) erreicht.

### Beispiel 3

Um die Sättigungskonzentration von Gemcitabin in wässrig-ethanolischen Lösungen zu ermitteln, wurden Gemcitabin-Lösungen mit verschiedenen Volumenanteilen an Ethanol hergestellt und analysiert.

Hierzu wurde Gemcitabinhydrochlorid im Ansatzbehälter vorgelegt, mit einer stöchiometrischen Menge an wässrig gelöstem Alkalisierungsmittel (1 N Natronlauge) versetzt, anschließend mit jeweils 80 % der in Vortests ermittelten erforderlichen Mengen an Wasser für Injektionszwecke als auch mit jeweils erforderlichen Mengen an Ethanol versetzt.

Die im Ansatz enthaltenen Bestandteile wurden anschließend unter 6-stündigem Rühren bei Raumtemperatur (19-23 °C) derart gelöst, dass ein ungelöster Bodensatz an Gemcitabinhydrochlorid im Ansatzbehälter verblieb. Der pH-Wert des Ansatzes wurde potentiometrisch bestimmt und erforderlichenfalls mit 1 N Natronlauge bzw. 1 N Salzsäure auf einen pH-Wert von 10 eingestellt, um mit diesem pH-Wert eine vollständige Umsetzung des Gemcitabinhydrochlorids zur gelösten Gemcitabinbase zu erzielen.

Der Ansatz wurde 24 Stunden bei 19-23 °C gelagert. Der pH-Wert wurde anschließend kontrolliert und nur solche klaren Überstände der Ansätze weiterverarbeitet, deren pH-Werte 10 +/- 0,5 betrugen. Diese wurden dann partikelfrei filtriert und der Gehalt an Gemcitabin per HPLC bestimmt.

Die Ergebnisse dieser Untersuchungen sind in der nachfolgenden Tabelle 2 zusammengefast.

**Tabelle 2:**

| Gemcitabin-Sättigungskonzentrationen ethanolisch-wässriger Lösungen (19-23°C; pH = 9,5-10,5) | | | |
|---|---|---|---|
| Testlösung | Ethanolanteil [Vol%] | Gehalt Gemcitabin-HCl [mg/ml] | Gehalt Gemcitabin-Base [mg/ml] |
| 1 | 0 | 15,81 | 13,9 |
| 2 | 10 | 21,92 | 19,3 |
| 3 | 20 | 26,26 | 23,1 |
| 4 | 30 | 33,67 | 29,6 |
| 5 | 40 | 39,22 | 34,5 |
| 6 | 50 | 49,38 | 43,4 |
| 7 | 60 | 57,39 | 50,4 |
| 8 | 70 | 55,28 | 48,6 |
| 9 | 80 | 48,30 | 42,4 |
| 10 | 90 | 31,21 | 27,4 |
| 11 | 100 | 5,35 | 4,70 |

Die Interpolation (Polynom 6. Grades, R²> 0,997) der experimentell ermittelten Daten ergab ein Maximum der Gemcitabin-Löslichkeit bei ca. 66 Vol.% Ethanolanteil von 50 mg/ml Gemcitabin bzw. 55 mg/ml Gemcitabinhydrochlorid bei einem pH-Wert von 10 +/-0,5.

### Beispiel 4

Die maximale Löslichkeit von Gemcitabinhydrochlorid wurde unter weiterer Verwendung von 1 N Natronlauge, Wasser für Injektionszwecke und absolutem Ethanol, in als hierfür optimal erkanntem 66 Vol.-%-igem Anteil bei gesteigerten Lösungstemperaturen (35-45 °C, 6 Stunden Rühren) und anschließendem Abkühlen auf Raumtemperatur (19-23 °C) und 24-stündiger Lagerung bei 19-23 °C ermittelt. Hierbei wurde im partikelfreien Überstand der gelagerten Lösungen mittels HPLC ein Gehalt von 95,1 mg/ml Gemcitabin bzw. 108,3 mg/ml Gemcitabinhydrochlorid bestimmt.

### Beispiel 5

Die Stabilität von Gemcitabin in ethanolisch-wässrigen Lösungskonzentraten wurde anhand von Gemcitabin-Lösungskonzentraten mit nachfolgend aufgeführten Zusammensetzungen (Testlösungen 12-21) ermittelt. Die "Stabilität der Lösung" ist immer als Stabilität des Gemcitabins in Lösung zu verstehen, d.h. als langfristige Konstanz der Konzentration der Ausgangsverbindung nach Inlösungbringen.

Zur Herstellung der in Tabelle3 genannten Lösungen wurde das Wasser für Injektionszwecke mit 90 % der erforderlichen Menge vorgelegt und Ethanol absolut als ein weiteres Lösungsmittel mit 90 % der erforderlichen Menge zugefügt. Der Ansatz wurde erwärmt auf 35-45 °C. 95 % der erforderlichen Menge an entsprechendem Alkalisierungsmittel (beispielsweise 1 N Natronlauge) wurden hinzugefügt und der Ansatz auf 35-45 °C erwärmt.

Das Gemcitabinhydrochlorid wurde mit 100 % der erforderlichen Menge hinzugefügt und unter Rühren klar gelöst, wobei der pH-Wert potentiometrisch bestimmt und mit der erforderlichen Menge des entsprechenden Alkalisierungsmittels (beispielsweise 1 N Natronlauge) auf den erwünschten pH-Wert eingestellt wurde.

Der Ansatz wurde mit der erforderlichen Menge an Wasser für Injektionszwecke und dem weiteren Lösungsmittel (Ethanol absolut) aufgefüllt. Der auf 35-45 °C erwärmte Ansatz wurde bis zum Erhalt einer klaren Lösung gerührt.

Der pH-Wert wurde nochmals kontrolliert und erforderlichenfalls mit Natronlauge oder Salzsäure auf den gewünschten pH-Wert eingestellt.

Der Ansatz wurde auf Raumtemperatur (19-23 °C) abgekühlt und in eine sterile Vorlage sterilfiltriert.

Das erhaltene Lösungskonzentrat wurde aseptisch in Durchstechflaschen (5 ml/Flasche) abgefüllt, diese mit Durchstechstopfen verschlossen und verbördelt.

Die Durchstechflaschen wurden lichtgeschützt bei 25 °C und "unter beschleunigten Bedingungen" bei 40 °C lichtgeschützt gelagert.

Zum jeweiligen Prüftakt wurden Muster der Analyse auf Gehalt und Reinheit (mittels HPLC) und pH-Wert (potentiometrisch) zugeführt.

Die Analyse der Stabilitätstaktprüfmuster ergab folgende in Tabelle 3 zusammengefasste Ergebnisse.

**Tabelle 3:**

| Stabilität und Zusammensetzungen der Testlösungen 12-21 | | | | | | | | | | | |
|---|---|---|---|---|---|---|---|---|---|---|---|
| Testlösung | | 12 | 13 | 14 | 15 | 16 | 17 | 18 | 19 | 20 | 21 |
| Gemcitabin (mg/ml) | | 50 | 50 | 80 | 40 | 30 | 40 | 30 | 25 | 80 | 60 |
| Natronlauge 1 N / Salzsäure 1 N | | - | q.s. | q.s. | - | - | q.s. | q.s. | q.s. | q.s. | q.s. |
| Dinatriumhydrogenphosphat-(H₂O)₂ [mg/ml] | | - | - | - | - | - | - | - | - | - | - |
| Trometamol 5 N | | - | - | - | q.s. | - | - | - | - | - | - |
| Meglumin 5 N | | q.s. | - | - | - | q.s. | - | - | - | - | - |
| Ethanol (in Vol%) | | 50 | 50 | 60 | 40 | 50 | 50 | 50 | 40 | 66 | 50 |
| Wasser für Injekt.-zwecke | | q.s. | q.s. | q.s. | q.s. | q.s. | q.s. | q.s. | q.s. | q.s. | q.s. |
| | pH-Wert | 7,0 | 8.4 | 8.0 | 8.9 | 8,0 | 8,2 | 8,1 | 8,8 | 7,6 | 8,4 |
| 25°C | Start | 100,0 | 100,0 | 100,0 | 100,0 | 100,0 | 100,0 | 100,0 | 100,0 | 100,0 | 100,0 |
| Gehalt (rel%) | 3 mon | 99,83 | 99,70 | 99,51 | 99,64 | 99,44 | 99,54 | 99,57 | 99,84 | 99,83 | 99,85 |
| | 6 mon | 99,59 | 99,61 | 99,54 | 99,34 | 99,78 | 99,24 | 99,34 | 99,51 | 99,67 | 99,70 |
| | 9 mon | 99,60 | 99,62 | 99,63 | 99,18 | 99,68 | 99,63 | 99,62 | 99,51 | 99,49 | 99,55 |
| | 12 mon | 99,47 | 99,51 | 99,36 | 99,10 | 98,57 | 99,52 | 99,49 | 99,40 | 99,33 | 99,43 |
| 40°C | Start | 100,0 | 100,0 | 100,0 | 100,0 | 100,0 | 100,0 | 100,0 | 100,0 | 100,0 | 100,0 |
| Gehalt (rel%) | 3 mon | 99,41 | 99,02 | 99,11 | 99,59 | 98,81 | 99,28 | 98,54 | 98,82 | 98,82 | 98,95 |
| | 6 mon | 98,60 | 99,21 | 99,04 | 98,74 | 97,58 | 98,67 | 97,25 | 97,63 | 98,05 | 97,69 |
| | 9 mon | 97,05 | 96,33 | 96,74 | 95,11 | 96,48 | 97,83 | 95,81 | 96,59 | 96,87 | 96,79 |
| | 12 mon | 95,83 | 94,98 | 95,52 | 93.65 | 95,32 | 97,28 | 94,30 | 95,44 | 95,99 | 95,52 |

Die Ergebnisse der Stabilitätsuntersuchungen an ethanolisch-wässrigen Gemcitabin-Lösungskonzentraten zeigen eindrucksvoll, dass Gemcitabin in den ethanolisch-wässrigen Konzentraten eine unerwartet hohe Lagerstabilität aufweist.

### Beispiel 6

Zur Ermittlung der Lagertemperatur-Abhängigkeit der Stabilität von gepufferten Gemcitabin-Lösungskonzentraten wurden Gemcitabin-Lösungskonzentrate mit den nachfolgenden, Dinatriumhydrogenphosphat-Dihydrat-gepufferten Zusammensetzungen (Testlösungen 22-27), wie in Beispiel 3 beschrieben, hergestellt und analysiert. Die Ergebnisse dieser Untersuchungen sind in der nachfolgenden Tabelle 4 zusammengefasst.

**Tabelle 4:**

| Stabilität der Phosphat-gepufferten Testlösungen 22-27 | | | | | | | |
|---|---|---|---|---|---|---|---|
| Testlösung | | 22 | 23 | 24 | 25 | 26 | 27 |
| Gemcitabin (mg/ml) | | 30 | 25 | 50 | 25 | 50 | 60 |
| Natronlauge 1 N / Salzsäure 1 N | | q.s. | q.s. | q.s. | q.s. | q.s. | q.s. |
| Dinatriumhydrogenphosphat-(H₂O)₂ [mg/ml] | | 2 | 2 | 2 | 4 | 4 | 4 |
| Trometamol 5 N | | - | - | - | - | - | - |
| Meglumin 5 N | | - | - | - | - | - | - |
| Ethanol (in Vol%) | | 50 | 50 | 50 | 40 | 66 | 50 |
| Wasser (Injektionszwecke) | | q.s. | q.s. | q.s. | q.s. | q.s. | q.s. |
| | pH-Wert | 7,5 | 7,2 | 7,1 | 7,8 | 7,6 | 7,4 |
| 25°C | Start | 100,0 | 100,0 | 100,0 | 100,0 | 100,0 | 100,0 |
| Gehalt (rel%) | 3mon | 99,54 | 99,63 | 99,47 | 99,74 | 99,87 | 99,89 |
| | 6mon | 99,82 | 99,34 | 99,34 | 99,61 | 99,57 | 99,71 |
| | 9mon | 99,63 | 99,53 | 99,22 | 99,54 | 99,59 | 99,45 |
| | 12mon | 98,51 | 99,42 | 99,19 | 99,13 | 99,23 | 99,53 |
| 40°C | Start | 100,0 | 100,0 | 100,0 | 100,0 | 100,0 | 100,0 |
| Gehalt (rel%) | 3mon | 98,71 | 99,28 | 98,58 | 98,72 | 98,73 | 98,65 |
| | 6mon | 97,68 | 98,56 | 97,35 | 97,53 | 98,15 | 97,67 |
| | 9mon | 96,53 | 97,73 | 95,71 | 96,79 | 96,59 | 96,73 |
| | 12mon | 95,42 | 96,68 | 94,40 | 95,94 | 96,11 | 95,62 |

Wie den in Tabelle 4 zusammengestellten Ergebnisse zu entnehmen blieben auch die gepufferten Lösungskonzentrate über die Dauer der Lagerungszeit ohne Auskristallisation partikelfrei und klar. Die Ergebnisse der bei Lagertemperaturen von 25 °C und 40 °C durchgeführten Stabilitätsuntersuchungen zeigen, dass sowohl ungepufferte als gepufferte, erfindungsgemäße Lösungskonzentrate selbst bei 40 °C-Lagerungstemperatur stabil sind.

### Beispiele 7-34

Es wurden wässrige Lösungskonzentrate folgende Zusammensetzung hergestellt:

**Tabelle 5:**

| Beispielhafte, erfindungsgemäße Gemcitabin- Lösungskonzentrate | | | | | |
|---|---|---|---|---|---|
| Bsp. | Gehalt Gemcitabin (mg/mL) | Weiteres Lösungsmittel (V/V) resp. Solubilisierungsmittel (m/V) | pH-Wert | Alkalisierungs-/ Ansäuerungsmittel resp. Puffer (m/V) | Weitere Hilfsstoffe (m/V) |
| 7 | 20,0 | 30 Vol.-% PEG 400 | 8,2 | Natronlauge/Salzsäure | |
| 8 | 20,0 | 40 Vol.-% PEG 400 | 7,6 | Natronlauge/Salzsäure | 0,13% (m/V) Methylparaben, 0,02% (m/V) Propylparaben |
| 9 | 20,0 | 30 Vol.-% PEG 400 | 7,0 | Natronlauge/Salzsäure | |
| 10 | 20,0 | 40% (m/V) Harnstoff | 7,0 | Natronlauge/Salzsäure | |
| 11 | 20,0 | 40% (m/V) Harnstoff | 7,2 | Natronlauge/Salzsäure 0,02 % (m/V) Natriumdihydrogenphosphat | |
| 12 | 30,0 | 40 Vol.-% Ethanol | 10,0 | Natronlauge/Salzsäure | |
| 13 | 30,0 | 40 Vol.-% Ethanol | 10,0 | Natronlauge/Salzsäure 0,1 % (m/V) Meglumin | |
| 14 | 30,0 | 40 Vol.-% Ethanol | 9,0 | Natronlauge/Salzsäure | |
| 15 | 30,0 | 40 Vol.-% Ethanol | 9,0 | Natronlauge/Salzsäure 0,1 % (m/V) Meglumin | |
| 16 | 30,0 | 40 Vol.-% Ethanol | 8,0 | Natronlauge/Salzsäure | |
| 17 | 30,0 | 40 Vol.-% Ethanol | 8,3 | Natronlauge/Salzsäure 0,1 % (m/V) TRIS | |
| 18 | 30,0 | 40 Vol.-% Ethanol | 7,6 | Natronlauge/Salzsäure 0,1 % (m/V) TRIS | |
| 19 | 20,0 | 50 Vol.-% Ethanol | 5,1 | Natronlauge/Salzsäure 0,025 % (m/V) Natriumacetat | |
| 20 | 40,0 | 50 Vol.- % Ethanol | 6,0 | Natronlauge/Salzsäure | |
| 21 | 40,0 | 50 Vol.-% Ethanol | 8,0 | Natronlauge/Salzsäure | |
| 22 | 50,0 | 50 Vol.-% Ethanol | 9,1 | Natronlauge/Salzsäure | |
| 23 | 50,0 | 50 Vol.-% Ethanol | 7,5 | Natronlauge/Salzsäure | |
| 24 | 50,0 | 50 Vol.-% Ethanol | 6,8 | Natronlauge/Salzsäure | |
| 25 | 50,0 | 50 Vol.-% Ethanol | 6,8 | Natronlauge/Salzsäure | 1,0% (m/V) Benzylalkohol |
| 26 | 20,0 | 60 Vol.-% Ethanol | 5,2 | Natronlauge/Salzsäure 0,025 % (m/V) Natriumacetat | |
| 27 | 20,0 | 60 Vol.-% Ethanol | 5,2 | Natronlauge/Salzsäure 0,025 % (m/V) Natriumacetat | 1,0% (m/V) Benzylalkohol |
| 28 | 80,0 | 60 Vol.-% Ethanol | 8,8 | Natronlauge/Salzsäure 0,1 % (m/V) TRIS | |
| 29 | 80,0 | 60 Vol.-% Ethanol | 7,8 | Natronlauge/Salzsäure | |
| 30 | 80,0 | 60 Vol.-% Ethanol | 7,8 | Natronlauge/Salzsäure | 0,13% (m/V) Methylparaben, 0,02% (m/V) Propylparaben |
| 31 | 80,0 | 60 Vol.-% Ethanol | 8,9 | Natronlauge/Salzsäure | |
| 32 | 20,0 | 30 Vol.-% Ethanol | 3,5 | Natronlauge/Salzsäure 0,025 % (m/V) Natriumlactat | |
| 33 | 20,0 | 30 Vol.-% Ethanol | 4,0 | Natronlauge/Salzsäure 0,025 % (m/V) Natriumlactat | |
| 34 | 20,0 | 30 Vol.-% Ethanol | 4,0 | Natronlauge/Salzsäure 0,025 % (m/V) Natriumazetat | |

## Patentansprüche

1. Pharmazeutische Zusammensetzung umfassend Gemcitabin in der Form eines gebrauchsfertigen Lösungskonzentrates, wobei das Gemcitabin in einem Gemisch aus Wasser und mindestens einem weiteren physiologisch annehmbaren Lösungsmittel bzw. Solubilisierungsmittel gelöst ist.

2. Pharmazeutische Zusammensetzung nach Anspruch 1, wobei das Gemcitabin-Lösungskonzentrat nicht unmittelbar vor der Verabreichung an den Patienten aus einem Feststoff rekonstituiert wurde.

3. Pharmazeutische Zusammensetzung nach Anspruch 1 oder 2, wobei als weiteres physiologisch annehmbares Lösungsmittel Ethanol, Polyethylenglykol 200-600 und/oder 1,2-Propandiol (Propylenglykol) bzw. als weiteres Solubilisierungsmittel Harnstoff verwendet wird.

4. Pharmazeutische Zusammensetzung nach einem der Ansprüche 1 bis 3, wobei die Gemcitabin-Konzentration zwischen 16,00 mg/ml und 110 mg/ml liegt.

5. Pharmazeutische Zusammensetzung nach Anspruch 4, wobei die Gemcitabin-Konzentration 50 mg/ml oder 80 mg/ml beträgt.

6. Pharmazeutische Zusammensetzung nach einem der Ansprüche 1-5, wobei der pH-Wert in einem Bereich von ca. 3,5 bis ca. 10,0 liegt.

7. Pharmazeutische Zusammensetzung nach Anspruch 6, wobei der pH-Wert der Lösung mit dem physiologischen Gewebe- und Blut-pH-Wert von 7,35 bis 7,55 übereinstimmt.

8. Pharmazeutische Zusammensetzung nach Anspruch 6 oder 7, wobei die Einstellung des pH-Wertes des Konzentrates durch Mischen der Gemcitabin-Base mit einem physiologisch annehmbaren Säureadditionssalz davon, vorzugsweise mit Gemcitabinhydrochlorid, erfolgt.

9. Pharmazeutische Zusammensetzung nach Anspruch 6 oder 7, wobei die Einstellung des pH-Wertes des Konzentrates mit mindestens einem physiologisch annehmbaren Ansäuerungsmittel erfolgt.

10. Pharmazeutische Zusammensetzung nach Anspruch 9, wobei das Ansäuerungsmittel Salzsäure ist.

11. Pharmazeutische Zusammensetzung nach Anspruch 6 oder 7, wobei die Einstellung des pH-Wertes des Konzentrates mit mindestens einem physiologisch annehmbaren Alkalisierungsmittel erfolgt.

12. Pharmazeutische Zusammensetzung nach Anspruch 11, wobei das Alkalisierungsmittel Natriumhydroxid ist.

13. Pharmazeutische Zusammensetzung nach Anspruch 6 oder 7, wobei die Einstellung des pH-Wertes des Konzentrates mit einem Puffer erfolgt.

14. Pharmazeutische Zusammensetzung nach Anspruch 13, wobei als Puffer Tris(hydroxymethyl-)aminomethan, 1-Desoxy-(methylamino)-D-glucitol, Natriumacetat und/oder Dinatriumhydrogenphosphat verwendet wird.

15. Pharmazeutische Zusammensetzung nach einem der Ansprüche 1-14, die gegebenenfalls zusätzlich mindestens einen tonisierenden Hilfsstoff, konservierenden Hilfsstoff und/oder ein Antioxidationsmittel umfasst.

16. Verwendung der pharmazeutischen Zusammensetzung nach einem der Ansprüche 1-15 zur Herstellung einer Lösung zur Verabreichung auf parenteralem Weg.

17. Verwendung der pharmazeutischen Zusammensetzung nach einem der Ansprüche 1 - 15 zur Herstellung eines Arzneimittels zur Behandlung einer Tumorerkrankung.

## Claims

1. Pharmaceutical composition including gemcitabine in the form of a ready-to-use solution concentrate, wherein the gemcitabine is dissolved in a mixture of water and at least one other physiologically acceptable solvent or solubilising agent.

2. Pharmaceutical composition according to claim 1, wherein the gemcitabine solution concentrate has not been reconstituted from a solid immediately before administration to the patients.

3. Pharmaceutical composition according to claim 1 or 2, wherein ethanol, polyethylene glycol 200 - 600 and/or 1,2-propanediol (propylene glycol) is used as another physiologically acceptable solvent and urea as another solubilising agent.

4. Pharmaceutical composition according to one of claims 1 to 3, wherein the gemcitabine concentration is between 16.00 mg/ml and 110 mg/ml.

5. Pharmaceutical composition according to claim 4, wherein the gemcitabine concentration is 50 mg/ml or 80 mg/ml.

6. Pharmaceutical composition according to one of claims 1 - 5, wherein the pH is in a range of approx. 3.5 to approx. 10.0.

7. Pharmaceutical composition according to claim 6, wherein the pH of the solution corresponds with the physiological tissue and blood pH of 7.35 to 7.55.

8. Pharmaceutical composition according to claim 6 or 7, wherein the pH of the concentrate is adjusted by mixing the gemcitabine base with a physiologically acceptable acid addition salt thereof, preferably with gemcitabine hydrochloride.

9. Pharmaceutical composition according to claim 6 or 7, wherein the pH of the concentrate is adjusted with at least one physiologically acceptable acidification agent.

10. Pharmaceutical composition according to claim 9, wherein the acidification agent is hydrochloric acid.

11. Pharmaceutical composition according to claim 6 or 7, wherein the pH of the concentrate is adjusted with at least one physiologically acceptable alkalisation agent.

12. Pharmaceutical composition according to claim 11, wherein the alkalisation agent is sodium hydroxide.

13. Pharmaceutical composition according to claim 6 or 7, wherein the pH of the concentrate is adjusted with a buffer.

14. Pharmaceutical composition according to claim 13, wherein tris(hydrbxymethyl-)aminomethane, 1-desoxy-(methylamino)-D-glucitol, sodium acetate and/or disodium hydrogen phosphate is used as buffer.

15. Pharmaceutical composition according to one of claims 1 - 14, which optionally includes in addition at least one toning aid, preservative aid and/or antioxidant.

16. Use of the pharmaceutical composition according to one of claims 1 - 15 for the production of a solution for administration in a parenteral way.

17. Use of the pharmaceutical composition according to one of claims 1 - 15 for the production of a pharmaceutical for the treatment of a tumour.

## Revendications

1. Composition pharmaceutique comprenant de la gemcitabine sous la forme d'un concentré de solution prêt à l'emploi, dans laquelle la gemcitabine est dissoute dans un mélange d'eau et d'au moins un solvant ou agent solubilisant supplémentaire acceptable sur le plan physiologique.

2. Composition pharmaceutique selon la revendication 1, dans laquelle le concentré de solution de gemcitabine n'a pas été reconstitué directement à partir d'un solide avant l'administration aux patients.

3. Composition pharmaceutique selon la revendication 1 ou 2, dans laquelle on emploie en tant que solvant supplémentaire acceptable sur le plan physiologique, l'éthanol, le polyéthylèneglycol 200-600 et/ou le 1,2-propanediol (propylèneglycol) ou en tant qu'agent solubilisant supplémentaire, l'urée.

4. Composition pharmaceutique selon l'une quelconque des revendications 1 à 3, dans laquelle la concentration en gemcitabine est comprise entre 16,00 mg/ml et 110 mg/ml.

5. Composition pharmaceutique selon la revendication 4, dans laquelle la concentration en gemcitabine est de 50 mg/ml ou 80 mg/ml.

6. Composition pharmaceutique selon l'une quelconque des revendications 1 à 5, dans laquelle la valeur de pH se trouve dans une plage d'environ 3,5 à environ 10,0.

7. Composition pharmaceutique selon la revendication 6, dans laquelle la valeur de pH de la solution coïncide avec la valeur physiologique du pH tissulaire et sanguin de 7,35 à 7,55.

8. Composition pharmaceutique selon la revendication 6 ou 7, dans laquelle l'ajustement de la valeur de pH du concentré se réalise en mélangeant la base de gemcitabine avec un sel d'addition d'acide acceptable sur le plan physiologique de celle-ci, de préférence avec du chlorhydrate de gemcitabine.

9. Composition pharmaceutique selon la revendication 6 ou 7, dans laquelle l'ajustement de la valeur de pH du concentré se réalise avec au moins un agent d'acidification acceptable sur le plan physiologique.

10. Composition pharmaceutique selon la revendication 9, dans laquelle l'agent d'acidification est l'acide chlorhydrique.

11. Composition pharmaceutique selon la revendication 6 ou 7, dans laquelle l'ajustement de la valeur de pH du concentré se réalise avec au moins un agent d'alcalinisation acceptable sur le plan physiologique.

12. Composition pharmaceutique selon la revendication il, dans laquelle l'agent d'alcalinisation est l'hydroxyde de sodium.

13. Composition pharmaceutique selon la revendication 6 ou 7, dans laquelle l'ajustement de la valeur de pH du concentré se réalise avec un tampon.

14. Composition pharmaceutique selon la revendication 13, dans laquelle on emploie comme tampon le tris(hydxoxyméthyl)aminométhane, le 1-désoxy-(méthylamino)-D-glucltol, l'acétate de sodium, et/ou l'hydrogénophosphate disodique.

15. Composition pharmaceutique selon l'une quelconque des revendications 1 à 14, qui comprend éventuellement en plus au moins un adjuvant de tonicité, un adjuvant conservateur et/ou un agent antioxydant.

16. Utilisation de la composition pharmaceutique selon l'une quelconque des revendications 1 à 15, pour préparer une solution destinée à l'administration par voie parentérale.

17. Utilisation de la composition pharmaceutique selon l'une quelconque des revendications 1 à 15, pour préparer un médicament destiné au traitement d'une maladie tumorale.
